(19) Europäisches Patentamt
European Patent Office
Office européen des brevets

(11) Numéro de publication: 0 140 748
B1

(12) FASCICULE DE BREVET EUROPÉEN

(45) Date de publication du fascicule du brevet: 14.01.87

(51) Int. Cl.⁴: C 07 D 215/22, C 07 C 125/03 // C07C101/447

(21) Numéro de dépôt: 84401861.4

(22) Date de dépôt: 20.09.84

# (54) Procédé de préparation de quinolinones-4.

(30) Priorité: 22.09.83 FR 8315073

(43) Date de publication de la demande: 08.05.85 Bulletin 85/19

(45) Mention de la délivrance du brevet: 14.01.87 Bulletin 87/3

(84) Etats contractants désignés: AT BE CH DE FR GB IT LI LU NL SE

(56) Documents cité:
EP-A-0 056 763
FR-A-2 312 491
FR-A-2 487 346
US-A-4 013 662

(73) Titulaire: RHONE- POULENC SANTE, Les Miroirs 18 Avenue d'Alsace, F-92400 Courbevoie Cedex (FR)

(72) Inventeur: Arnaud, Michel, 31, rue Boileau, F-69006 Lyon (FR)
Inventeur: Corbet, Jean- Pierre, "Les Marronniers" Résidence "Charrière Blanche", F-69130 Ecully (FR)

(74) Mandataire: Pilard, Jacques, RHONE- POULENC RECHERCHES Service Brevets Pharma 25, Quai Paul Doumer, F-92408 Courbevoie Cedex (FR)

EP 0 140 748 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La présente invention concerne un nouveau procédé de préparation de tétrahydro-1,2,3,4 quinolinones-4 de formule générale:

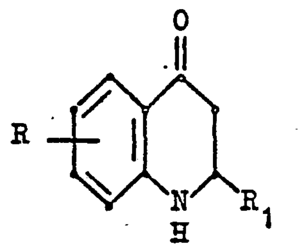

(I)

qui sont particulièrement utiles comme intermédiaires dans la synthèse de substances thérapeutiquement actives.

Dans la formule générale (I), R représente un atome d'hydrogène ou un substituant choisi dans le groupe des atomes d'halogène et des radicaux alcoyles droits ou ramifiés contenant 1 à 4 atomes de carbone et alcoyloxy droits ou ramifiés contenant 1 à 4 atomes de carbone, et $R_1$ représente un atome d'hydrogène ou radical alcoyle droit ou ramifié contenant 1 à 4 atomes de carbone.

Il est connu de préparer des tétrahydro-1,2,3,4 quinolinones-4 de formule générale (I) par cyclisation d'un acide anilino-3 propionique au moyen d'acide polyphosphorique selon le procédé décrit dane le brevet français FR 1 514 280, au moyen d'un oléum selon le procédé décrit dans le brevet européen EP 56 764 ou encore au moyen d'un mélange acide fluorhydrique-trifluorure de bore selon le procédé décrit dans le brevet européen EP 56 763.

Il faut cependant remarquer que, dans le cas particulier de la cyclisation de l'acide m.chloroanilino-3 propionique par l'acide polyphosphorique, un mélange en quantités sensiblement égales de chloro-5 et de chloro-7 tétrahydro-1,2,3,4 quinolinone -4 est obtenu. La sélectivité en chloro-7 tétrahydro-1,2,3,4 quinolinone-4 est notablement améliorée si on utilise comme agent de cyclisation un oléum ou un mélange acide fluorhydriquetrifluorure de bore mais la mise en oeuvre industrielle de ces procédés se heurte à des difficultés résultant soit de la manipulation de quantités importantes d'acide sulfurique soit de l'utilisation d'un mélange d'acide fluorhydrique anhydre et de trifluorure de bore.

Il a maintenant été trouvé, et c'est ce qui fait l'objet de la présente invention, que les produits de formule générale (I) peuvent être obtenus avec des bons rendements, et lorsque le cas se présente, avec une sélectivité accrue, à partir d'acides anilino-3 propioniques par la mise en oeuvre de réactions plus simples et aussi performantes que celles antérieurement connues.

Selon l'invention, un acide anilino-3 propionique de formule générale:

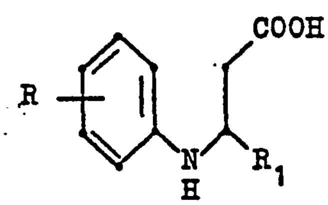

(II)

dans laquelle R et $R_1$ sont définis comme précédemment, est traité par le phosgène pour obtenir un produit de formule générale:

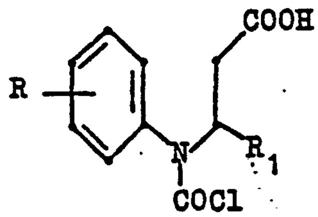

(III)

dans laquelle R et $R_1$ sont définis comme précédemment, qui, sous l'action du phosgène en présence de diméthylformamide, est transformé en chlorure d'acide de formule générale:

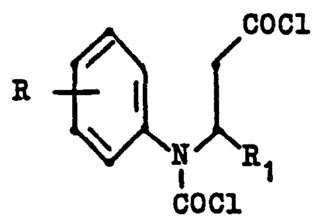

(IV)

dans laquelle R et $R_1$ sont définis comme précédemment, qui, par action d'un acide de Lewis dans un solvant organique convenable, est cyclisé en produit de formule générale:

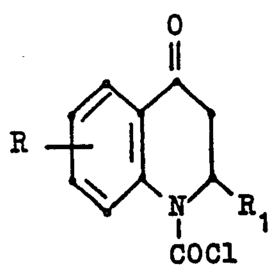

(V)

dans laquelle R et $R_1$ sont définis comme précédemment, qui est ensuite hydrolysé par action d'une base en produit de formule générale (I).

Selon l'invention, les produits de formule générale (III) sont obtenus par action du phosgène sur un produit de formule générale (II) en opérant dans un solvant organique tel que le chlorure de méthylène, le trichloro-1,1,2 éthane, les esters aliphatiques ou le dioxanne à une température comprise entre -10 et 150°C et, de préférence entre -30 et 80°C. Généralement, on utilise un léger excès de phosgène (inférieur à 10%) par rapport à l'acide anilino-3 propionique de formule générale (II) mis en oeuvre. Il est avantageux d'opérer en atmosphère inerte.

Selon l'invention, les produits de formule générale (IV) sont obtenus par action du phosgène sur les produits de formule générale (III) en présence d'une trace de diméthylformamide en opérant dans un solvant organique tel que le chlorure de méthylène, les trihalogénoéthanes tels que le trichloro-1,1,2 éthane ou le dioxanne à une température comprise entre -10 et 100°C et de préférence entre -0 et 50°C. Généralement, on utilise un léger excès de phosgène (inférieur à 10%) par rapport au produit de formule générale (III). Il est avantageux d'opérer en atmosphère inerte.

Selon l'invention, les produits de formule générale (V) sont obtenus par cyclisation intramoléculaire d'un produit de formule générale (IV) au moyen d'un acide de Lewis ou d'un acide fort.

Comme acides de Lewis, conviennent particulièrement bien le chlorure d'aluminium, le chlorure ferrique, le chlorure de titane ou le chlorure stannique.

Comme acides forts, conviennent particulièrement bien l'acide sulfurique, l'acide fluorhydrique, les acides

sulfoniques (acides alcoylsulfoniques, arylsulfoniques, trifluorométhanesulfoniques) ou l'acide polyphosphorique.

Lorsqu'on utilise un acide de Lewis, la réaction est mise en oeuvre dans un solvant convenablement choisi à une température comprise entre -10 et 70°C et de préférence entre 0 et 50°C. Comme solvants organiques peuvent être utilisés les hydrocarbures halogénés tels que le chlorure de méthylène, les trihalogénoéthanes tels que le trichloro-1,1,2 éthane, le sulfure de carbone, les nitroalcanes ou le tétrachloroéthylène.

Lorsqu'on utilise un acide fort, la cyclisation s'effectue à une température comprise entre 20 et 100°C.

Selon l'invention, les produits de formule générale (V) peuvent aussi être obtenus directement à partir d'un acide anilino-3 propionique sans qu'il soit nécessaire d'isoler intermédiairement les produits de formule générale (III) et (IV).

A cet effet, le produit de formule générale (II) est traité par le phosgène, à raison d'au moins 2 moles de phosgène par mole d'acide anilino-3 propionique de formule générale (II) mis en oeuvre, dans un solvant organique tel que le chlorure de méthylène ou le trichloro-1,1,2 éthane à une température comprise entre -10 et 150°C et de préférence entre 30 et 80°C, puis le diméthylformamide est ajouté et la réaction est poursuivie jusqu'à transformation complète. Après élimination de l'excès de phosgène, l'acide de Lewis ou l'acide fort est ajouté au mélange réactionnel et la réaction est poursuivie jusqu'à disparition totale du produit de départ et des produits intermédiaires. Lorsque la réaction est terminée, le produit de formule générale (V) est séparé du mélange réactionnel selon les méthodes habituelles.

Selon l'invention, les produits de formule générale (I) peuvent être obtenus par action d'une base minérale sur un produit de formule générale (V). Il est particulièrement avantageux d'utiliser la soude en solution méthanolique en opérant à une température comprise entre 50 et 100°C.

Les produits de formule générale (I) peuvent être isolés du mélange réactionnel et purifiés par application des méthodes habituelles.

Les produits de formule générale (IV) et les produits de formule générale (V), à l'exception de ceux pour lesquels $R_1$ représente un atome d'hydrogène et R représente un atome de chlore, de brome ou de fluor en position 6, sont des produits nouveaux qui constituent un autre objet de la présente invention.

Les acides de formule générale (II) utilisés comme produit de départ peuvent être obtenus par action d'une aniline convenablement substituée sur un acide de formule générale:

$R_1$-CH=CH-COOH (VI)

dans laquelle $R_1$ est défini comme précédemment.

La réaction s'effectue généralement dans l'eau à une température comprise entre 70 et 100°C en utilisant un excès d'aniline par rapport à l'acide de formule générale (VI) mis en oeuvre. La durée de la réaction est généralement comprise entre 1 et 4 heures.

Les tétrahydro-1,2,3,4 quinolinones-4 de formule générale (I) sont particulièrement utiles comme intermédiaires dans la synthèse de substances thérapeutiquement actives telles que la chloroquine, la glafénine, l'antrafénine ou l'amodiaquine.

Plus particulièrement, la chloro-7 tétrahydro-1,2,3,4 quinolinone-4 peut être transformée en chloroquine par condensation de la diéthylamino-4 méthyl-1 butylamine en présence d'air selon le procédé décrit par W.S. Johnson et B.G. Buell, J. Amer. Chem. Soc., 74, 4513 (1952).

Les exemples suivants, donnés à titre non limitatif, montrent comment l'invention peut être mise en pratique.

**Exemple 1**

Dans un ballon de 100 $cm^3$ muni d'une agitation magnétique, d'un thermomètre, d'un réfrigérant ascendant à acétone-carboglace, d'un dispositif d'introduction de gaz et d'une ampoule de coulée, on introduit 20 $cm^3$ de chlorure de méthylène sec. On condense, à une température comprise entre 0 et 5°C, 3,15 g (31,8 m.moles) de phosgène. Le mélange réactionnel est maintenu sous atmosphère d'argon. On ajoute ensuite, en 19 minutes, une solution de 6,25 g (31,3 m.moles) d'acide m.chloroanilino-3 propionique dans 15 $cm^3$ de chlorure de méthylène. La température monte de 6 à 23°C. Le mélange réactionnel est alors constitué d'une phase liquide jaune et d'un précipité blanc en suspension. On poursuit l'agitation pendant 10 minutes à 23°C puis on fait passer, pendant 50 minutes, un courant d'argon dans le mélange réactionnel pour éliminer le phosgène n'ayant pas réagi.

Le précipité est séparé par filtration sous courant d'argon puis séché sous pression réduite (1 mm de mercure; 0,13 kPa) jusqu'à poids constant. On obtient ainsi 3,2 g de chlorhydrate de l'acide m.chloroanilino-3 propionique.

Le filtrat est concentré à sec. On obtient ainsi 4,58 g d'acide N-chloroformyl m.chloroanilino-3 propionique, pratiquement pur, fondant à 106°C dont la structure est confirmée par le spectre infra-rouge, le spectre de masse et le spectre de résonance magnétique nucléaire du proton.

Le produit, après recristallisation dans l'éther isopropylique, fond à 111°C.

L'acide m.chloroanilino-3 propionique peut être préparé de la manière suivante:

A un mélange de 510,3 g de m.chloroaniline dans 150 $cm^3$ d'eau, maintenu sous atmosphère d'argon agité à 80°C, on ajoute en 10 minutes une solution de 72,05 g d'acide acrylique dans 100 $cm^3$ d'eau. Le mélange réactionnel, constitué de deux phases, est maintenu pendant 3 heures à 80°C sous agitation puis refroidi à

20° C. Après décantation, la phase aqueuse (couche supérieure) est éliminée. A la phase organique on ajoute 423 $cm^3$ d'une solution aqueuse de soude 2,6 N, en agitant et en maintenant la température à 20° C. Après décantation, la phase organique constituée de 303 g de m.chloroaniline est séparée. La phase aqueuse (850 $cm^3$) est extraite 6 fois successivement par 450 $cm^3$ d'éther.

La phase aqueuse, dont on élimine l'éther par évaporation sous pression réduite (20 mm de mercure; 2,7 kpa), est acidifiée par addition de 105 g d'acide sulfurique à 50% (en poids). Le pH final est 3,5 (point isoélectrique). La température passe de 22 à 33° C puis on chauffe à 40° C. Après décantation, on sépare:

- une phase organique inférieure (208,8 g) constituée d'acide m.chloroanilino-3 propionique fondu saturé d'eau (8,6 % d'eau)
- une phase aqueuse supérieure (601 g) contenant 2,28 g d'acide m.chloroanilino-3 propionique et 156 g de sulfate de sodium.

La phase organique est chauffée pendant 1 heure à 80° C sous pression réduite (20 mm de mercure; 2,7 kPa). On obtient 195,4 g d'un produit contenant 94 % d'acide m.chloro-anilino-3 propionique et 2,3 % d'eau.

**Exemple 2**

Dans un appareillage identique à celui utilisé dans l'exemple 1, on introduit 30 $cm^3$ de chlore de méthylène sec et 0,2 $cm^3$ de diméthylformamide. On condense ensuite, à une température voisine de 5°C, 3,2 g de phosgène (32,35 m.moles). Au cours de la condensation il se forme un léger précipité blanc. On laisse remonter la température vers 16° C puis on ajoute une solution de 7,06 g d'acide N-chloroformyl m.chloro-anilino-3 propionique (26,93 m.moles) dans 50 $cm^3$ de chlorure de méthylène sec. Le précipité se dissout progressivement et à la fin de l'addition le mélange réactionnel est jaune et limpide. La température du mélange réactionnel est de 27° C. On élimine l'excès de phosgène en faisant barboter un courant d'argon dans le mélange réactionnel pendant 30 minutes puis on laisse reposer pendant 15 heures à une température voisine de 20° C.

Le mélange réactionnel est concentré sous pression réduite (20 mm de mercure; 2,7 kPa) à 40° C. On obtient ainsi 8 g d'une huile jaune-orange. Cette huile est reprise par 20 $cm^3$ d'acétate d'éthyle à 4° C. Il se forme un léger précipité qui est séparé par filtration sous atmosphère d'argon. Le filtrat est concentré à sec sous pression réduite (20 mm de mercure; 2,7 kpa) puis séché à poids constant sous pression réduite (1 mm de mercure; 0,13 kpa) à 40° C. On obtient ainsi 7,4 g de chlorure d'acide de l'acide N-chloroformyl m. chloroanilino-3 propionique, pratiquement pur, sous forme d'huile orange, dont la structure est confirmée par l'analyse élémentaire quantitative, le spectre infrarouge, le spectre de masse et le spectre de résonance magnétique nucléaire du proton.

**Exemple 3**

Dans un appareillage identique à celui utilisé dans l'exemple 1, on introduit 25 $cm^3$ de chlorure de méthylène. On fait alors passer, pendant 10 minutes, un courant de phosgène à une température voisine de 20° C de façon à éliminer l'eau et l'éthanol présents dans le solvant utilisé. Le phosgène dissous est éliminé en faisant passer un courant d'argon pendant 20 minutes à une température voisine de 40°C. On ajoute alors 2 g de chlorure d'acide de l'acide N-chloroformyl m.chloroanilino-3 propionique (7,13 m.moles) en opérant sous atmosphère d'argon à une température de 26° C. On ajoute alors, en 10 minutes, 2,09 g de chlorure d'aluminium anhydre, la température passant de 26 à 32° C. Le mélange réactionnel est alors constitué d'une phase liquide jaune et d'une suspension. On laisse réagir pendant 15 heures à une température voisine de 20°C puis on verse le mélange réactionnel sur 25 g de glace. La phase organique est séparée par décantation. La phase aqueuse est extraite par 4 fois 25 $cm^3$ de chlorure de méthylène. Les extraits organiques rassemblés sont lavés par 4 fois 25 $cm^3$ d'eau puis séchés sur sulfate de sodium. Après filtration, le filtrat est concentré à sec sous pression réduite (20 mm de mercure; 2,7 kPa ) à 40°C. On obtient alors 1,5 g de chloroformyl-1 chloro-7 tétrahydro-1,2,3,4 quinolinone-4 fondant à 150-153° C qui, après recristallisation dans l'acétate d'éthyle, fond à 156° C.

La structure de la chloroformyl-1 chloro-7 tétra-hydro-1,2,3,4 quinolinone-4 est confirmée par son analyse élémentaire quantitative, son spectre infra-rouge, son spectre de masse et son spectre de résonance magnétique nucléaire du proton.

**Exemple 4**

Dans 20 $cm^3$ d'une solution normale de soude dans le méthanol, on introduit 0,5 g (2,05 moles) de chloroformyl-1 chloro-7 tétrahydro-1,2,3,4 quinolinone-4. On chauffe à 65° C pendant 3 heures 10 minutes. Le mélange réactionnel est concentré sous pression réduite (20 mm de mercure; 2,7 kpa) à 40° C, puis il est repris par 100 $cm^3$ d'eau et 100 $cm^3$ de chlorure de méthylène. Après décantation, la phase aqueuse est extraite par 3

fois 50 $cm^3$ de chlorure de méthylène. Les phases organiques réunies sont lavées par 4 fois 50 $cm^3$ d'eau distillée puis séchées sur sulfate de sodium. Après filtration et concentration à sec, on obtient 0,36 g de chloro-7 tétra-hydro-1,2,3,4 quinolinone-4 fondant à 132°C.

L'analyse de ce produit par chromatographie en phase vapeur montre qu'il contient 91 % de chloro-7 tétrahydro-1,2,3,4 quinolinone-4.

**Exemple 5**

Dans un appareillage identique à celui décrit dans l'exemple 1 on introduit 20 $cm^3$ de chlorure de méthylène sec. On condense ensuite entre 4 et 5°C, 7,85 g de phosgène (79,37 m.moles) puis on ajoute, en 15 minutes, une solution de 5,946 g d'acide m.chloroanilino-3 propionique (29,81 m.moles) dans 12 $cm^3$ de chlorure de méthylène. Pendant l'addition la température du mélange réactionnel passe de 7 à 17°C et il se fforme un précipité beige abondant. On chauffe alors à 30°C pendant 30 minutes. Après refroidissement à 25°C on ajoute, à l'aide d'une seringue, 0,1 $cm^3$ de diméthylformamide sec. Il se produit un dégagement gazeux régulier qui ralentit au bout de 30 minutes. On ajoute à nouveau 0,1 $cm^3$ de diméthylformamide: le dégagement gazeux reprend. Au bout d'une heure, le précipité initial est totalement dissous. La disparition totale du produit de départ est confirmée par chromatographie sur couche mince. Le mélange réactionnel est chauffé à 38°C puis on fait passer un courant d'argon pour éliminer l'excès de phosgène n'ayant pas réagi.

Après refroidissement à 20°C, on ajoute, en 40 minutes, 8,49 g de chlorure d'aluminium anhydre (63,66 m.moles). On laisse réagir à une température voisine de 20°C pendant 23 heures 45 minutes.

Le mélange réactionnel est versé sur 150 g de glace. La phase aqueuse est extraite par 4 fois 50 $cm^3$ de chlorure de méthylène et les phases organiques réunies sont lavées par 5 fois 30 $cm^3$ d'eau puis séchées sur sulfate de sodium anhydre. Après filtration et concentration à sec du filtrat sous pression réduite (20 mm de mercure; 2,7 kPa) à 40°C, on obtient 7,294 g de chloroformyl-1 chloro-7 tétrahydro-1,2,3,4 quinolinone-4, pratiquement pure, sous forme d'un solide jaune.

On traite 477,7 mg de chloroformyl-1 chloro-7 tétrahydro-1,2,3,4 quinolinone-4 par une solution normale de soude méthanolique dans les conditions décrites dans l'exemple 4. On obtient ainsi 363,4 mg d'un solide jaune clair dont l'analyse par chromatographie en phase vapeur et par chromatographie liquide haute performance montre qu'il contient 89 % de chloro-7 tétrahydro-1,2,3,4 quinolinone-4 et 0,5 % de chloro-5 tétrahydro-1,2,3,4 quinolinone-4.

Le rendement en chloro-7 tétrahydro-1,2,3,4 quino-linone-4 est de 91,2 % par rapport à l'acide m.chloroanilino-3 propionique mis en oeuvre.

**Exemple 6**

A une solution de 1,37 9 (4,90 m.moles) de chlorure de l'acide N-chloroformyl m.chloroanilino-3 propionioue dans le chlorure de méthylène (5,7 $cm^3$), préparée de manière identique 3 celle décrite dans l'exemple 2, on ajoute 2,04 g (10,77 m.moles) de tétrachlorure de titane en 10 minutes à une température voisine de 20°C. Le mélange réactionnel est agité à cette température pendant 20 heures puis à une température voisine de 40°C pendant 4 heures 30 minutes. Après refroidissement à une température voisine de 20°C le mélange réactionnel est versé sur 25 g de glace. La phase organique est séparée par décantation. La phase aqueuse est extraite par 4 fois 15 $cm^3$ de chlorure de méthylène. Les extraits organigues rassemblés sont lavés par deux fois 20 $cm^3$ d'eau puis séchés sur sulfate de sodium. Après filtration le filtrat est concentré à sec sous pression réduite (20 mm de mercure; 2,7 kPa) à 40°C. On obtient alors 1,195 g d'un résidu brun contenant essentiellement la chloroformyl-1 chloro-7 tétrahydro-1,2,3,4 quinolinone-4.

Une partie de ce résidu (0,460 g) est traité par 20 $cm^3$ d'une solution de soude N dans le méthanol d'une manière identique à celle décrite dans l'exemple 4. On obtient ainsi 0,170 g d'un résidu brun.

L'analyse de ce résidu par chromatographie en phase vapeur montre qu'il contient 81,5% de chloro-7 tétrahydro-1,2,3,4 quinolinone-4.

Le rendement est de 40,5% par rapport à l'acide m.chloroanili-no-3 propionique mis en oeuvre.

**Exemple 7**

Dans un réacteur en acier inoxydable contenant une solution de 1,26 g (4,5 m.moles) de chlorure de l'acide N-chloroformyl m-chloro-anilino-3 propionique dans 5 $cm^3$ de chlorure de méthylène refroidie à 0°C on introduit 20,0 g d'acide fluorhydrique anhydre. Le réacteur est alors fermé puis chauffé à 80°C pendant 17 heures 25 minutes. La pression intérieure est d'environ 6 bars. Le réacteur est ensuite refroidi à 10°C et le liquide est versé sur un mélange de 100 g d'eau et de glace. Après extraction par 3 fois de 30 $cm^3$ de chlorure de méthylène, les extraits organiques réunis sont lavés par 70 $cm^3$ d'une solution de carbonate de potassium

puis par 2 fois 50 $cm^3$ d'eau distillée et enfin séchés sur sulfate de sodium. Après filtration et concentration à sec sous pression réduite (20 mm de mercure; 2,7 kPa) on obtient 0,75 g d'un résidu huileux.

Ce résidu est traité par 30 $cm^3$ d'une solution normale de soude méthanolique d'une manière identique à celle décrite dans l'exemple 4. On obtient ainsi 0,40 g d'un résidu jaune semi-cristallin.

L'analyse de ce résidu par chromatographie en phase vapeur montre qu'il contient 77,9% de chloro-7 tétrahydro-1,2,3,4 quinolinone-4.

Le rendement est de 38,2% par rapport au chlorure de l'acide m-chloroanilino-3 propionique engagé.

**Exemple 8**

Dans un appareillage identique à celui décrit dans l'exemple 1 on introduit 6,27 g (35,0 m.moles) d'acide (méthyl-2 anilino)-3 propionique et 38 $cm^3$ de chlorure de méthylène sec. Le mélange réactionnel est maintenu au voisinage de 40°C et on introduit en 37 minutes 9,35 g (94,5 m.moles) de phosgène. Pendant cette addition, il se forme un précipité blanc. On ajoute 0,25 g (3,4 m.moles) de diméthylformamide sec è l'aide d'une seringue à une température voisine de 35°C. Il se produit un dégagement gazeux qui cesse au bout d'une heure environ tandis que la température du mélange réactionnel est maintenu entre 35°C et 40°C. On fait alors passer un courant d'argon pour chasser l'excès de phosgène n'ayant pas réagi.

Après refroidissement vers 20°C, on ajoute en 6 minutes 11,29 g (84,7 m.moles) de chlorure d'aluminium. On laisse réagir à une température voisine de 20°C pendant 18 heures 45 minutes.

Le mélange réactionnel est alors traité d'une manière analogue à celle décrite dans l'exemple 5. On obtient ainsi 7,83 g (35,0 m.moles) d'un produit jaune fondant à 99°C. L'analyse spectrale de ce produit montre qu'il s'agit de chloroformyl-1 méthyl-8 tétrahydro-1,2,3,4 quinolinone-4 pratiquement pure. Après recristallisation dans l'hexane le produit fond à 108°C.

L'acide (méthyl-2 anilino)-3 propionique est préparé d'une manière analogue à celle décrite dans l'exemple 1 mais à partir de 64,3 g (0,60 mole) d'o-toluidine, de 11,6 g (0,16 mole) d'acide acrylique et de 36,6 $cm^3$ d'eau. On obtient ainsi 23,85 g d'une poudre blanche qui, après recristallisation dans un mélange toluènehexane 70-30 (en volumes),donne 21,7 g (0 l2 mole) de produit pur fondant à 87°C.

**Example 9**

Dans un ballon de 50 $cm^3$ muni d'une agitation magnétique, d'un thermomètre, d'un réfrigérant et d'une ampoule de coulée on introduit 1,17 g (5,2 m.moles) de chloroformyl-1 méthyl-8 tétrahydro-1,2,3,4 quinolinone-4 et 5 $cm^3$ de xylène. Le mélange est alors chauffé au moyen d'un bain d'huile maintenu à 115°C et on ajoute 4 $cm^3$ de soude 5N . Après 2 heures 40 minutes le mélange réactionnel est refroidi à 20°C, et on ajoute 20 $cm^3$ de chlorure de méthylène et 20 $cm^3$ d'eau distillée. La phase aqueuse est séparée par décantation et extraite par 4 fois 10 $cm^3$ de chlorure de méthylène. Les extraits organiques rassemblés sont lavés par 2 fois 20 $cm^3$ d'eau puis séchés sur sulfate de sodium. Après filtration, le filtrat est concentré à sec sous pression réduite (20 mm de mercure; 2,7 kPa) à 40°C. On obtient 0,90 g d'un produit jaune fondant entre 86°C et 88°C. Le spectre de résonance magnétique nucléaire montre qu'il s'agit de méthyl-8 tétrahydro-1,2,3,4 quinolinone-4 contenant 10% d'impuretés. Après recristallisation dans l'hexane, le produit fond à 98°C.

La structure de la méthyl-8 tétrahydro-1,2,3,4 quinolinone-4 est confirmée par les spectres infra-rouge, de masse et de résonance magnétique nucléaire du proton.

**Exemple 10**

La chloro-7 chloroformyl-1 méthyl-2 tétrahydro-1,2,3,4 quino-linone-4 est préparée d'une manière analogue à celle décrite dans l'exemple 9 mais à partir de 5,42 g (25,4 m.moles) d'acide (chloro-3 anilino)-3 butanoïque, de 6,7 g (67,7 m.moles) phosgène, de 25 $cm^3$ de chlorure de méthylène, de 0,23 $cm^3$ de diméthylformamide et de 8,15 (61,1 m.moles) de chlorure d'aluminium.

On obtient ainsi 4,79 g d'un produit blanc fondant à 132°C. L'analyse des spectres infra-rouge, de masse et de résonance magnétique nucléaire montre qu'il s'agit de chloro-7 chloroformyl-1 méthyl-2 tétrahydro-1,2,3,4 quinolinone-4 pratiquement pure et dans laquelle on n'observe pas de chloro-5 chloroformyl-1 méthyl-2 tétrahydro-1,2,3,4 quinolinone-4.

Après recristallisation dans un mélange toluène-hexane 50-50 (en volumes) le produit fond à 134°C.

L'acide (chloro-3 anilino)-3 butanoïque est préparé d'une manière analogue à celle décrite dans l'exemple 1 mais à partir de 65 $cm^3$ (0,5 mole) de chloro-3 aniline, de 11,20 g (0,129 mole) d'acide crotonique et de 31 $cm^3$ d'eau distillée. On obtient ainsi après purification 14,66 g (68,6 m.moles) de produit sous forme d'une poudre blanche fondant vers 50°C.

**Exemple 11**

La chloro-7 méthyl-2 tétrahydro-1,2,3,4 quinolinone-4 est préparée d'une manière analogue à celle décrite dans l'exemple 4 mais à partir de 1,30 g (5,0 m.moles) de chloro-7 chloroformyl-1 méthyl-2 tétra-hydro-1,2,3,4 quinolinone-4 de 5 $cm^3$ de xylène et de 4 $cm^3$ de soude 5N.

On obtient ainsi 1,08 g d'un produit jaune fondant entre 155° C et 160° C. L'analyse des spectres infra-rouge, de masse et de résonance magnétique nucléaire montre qu'il s'agit de chloro-7 méthyl-2 tétrahydro-1,2,3,4 quinolinone-4 pratiquement pure dans laquelle on n'observe pas de chloro-5 méthyl-2 tétrahydro-1,2,3,4 quinolinone-4.

Après recristallisation dans un mélange hexane-toluène 50-50 (en volumes) le produit fond à 168° C.

**Revendications** pour les Etats contactants:

BE, CH, DE, FR, GB, IT, LI, LU, NL, SE

1 - Procédé de préparation d'une tétrahydro-1,2,3,4 quinolinone-4 de formule générale:

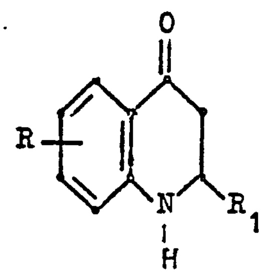

dans laquelle R représente un atome d'hydrogène ou un substituant choisi dans le groupe des atomes d'halogène et des radicaux alcoyles droits ou ramifiés contenant 1 à 4 atomes de carbone ou alcoyloxy droits ou ramifiés contenant 1 à 4 atomes de carbone et $R_1$ représente un atome d'hydrogène ou un radical alcoyle droit ou ramifié contenant 1 à 4 atomes de carbone caractérisé en ce que l'on traite par le phosgène un acide anilino-3 propionique de formule générale:

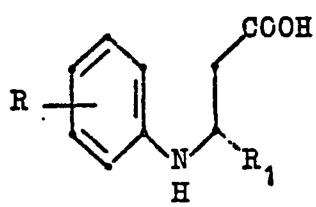

dans laquelle R et $R_1$ sont définis comme précédemment pour obtenir un produit de formule générale:

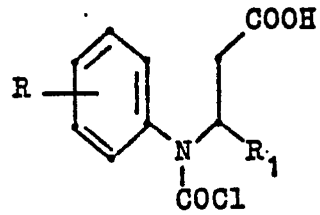

dans laquelle R et $R_1$ sont définis comme précédemment, que l'on transforme par action du phosgène en présence de diméthylformamide en chlorure d'acide de formule générale:

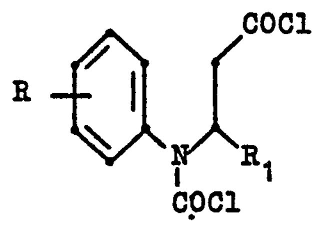

dans laquelle R et $R_1$ sont définis comme précédemment, que l'on traite par un acide de Lewis dans un solvant convenable ou par un acide fort pour obtenir un produit de formule générale:

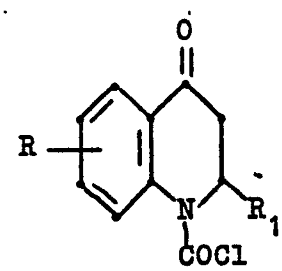

dans laquelle R et $R_1$ sont définis comme précédemment, que l'on hydrolyse en présence d'une base minérale puis isole la tétrahydro-1,2,3,4 quinolinone-4 ainsi obtenue.

2 - Procédé selon la revendication 1 caractérisé en ce que le traitement par le phosgène est effectué dans un solvant organique à une température comprise entre -10 et 150°C.

3 - Procédé selon la revendication 2 caractérisé en ce que le solvant est choisi parmi le chlorure de méthylène, le trichloro-1,1,2 éthane et le dioxanne.

4 - Procédé selon la revendication 1 caractérisé en ce que l'on utilise un acide de Lewis choisi parmi le chlorure d'aluminium, le chlorure ferrique, le chlorure de titane ou le chlorure stannique dans un solvant organique.

5 - Procédé selon la revendication 4 caractérisé en ce que le solvant organique est choisi parmi les hydrocarbures halogénés, le sulfure de carbone, les nitroalcanes, et le tétrachloroéthylène.

6 - Procédé selon la revendication 1 caractérisé en ce que l'on utilise comme acide fort l'acide sulfurique, l'acide fluorhydrique, les acides sulfoniques ou l'acide polyphosphorique.

7 - Procédé selon la revendication 1 caractérisé en ce que l'on utilise comme base la soude méthanolique.

8 - Procédé selon la revendication 1 caractérisé en ce que l'on prépare le produit de formule générale:

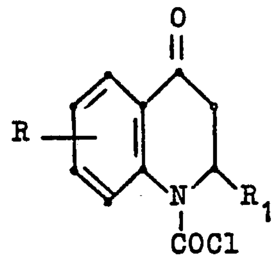

dans laquelle R et $R_1$ sont définis comme dans la revendication 1 sans isolement des produits intermédiaires.

9 - Le chlorure de l'acide N-chloroformyl anilino-3 propionique de formule générale:

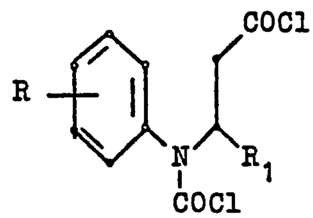

dans laquelle R représente un atome d'hydrogène ou un substituant choisi dans le groupe des atomes d'halogène et des radicaux alcoyles droits ou ramifiés contenant 1 à 4 atomes de carbone et alcoyloxy droits ou ramifiés contenant 1 à 4 atomes de carbone et $R_1$ représente un atome d'hydrogène ou un radical alcoyle droit ou ramifié conténant 1 à 4 atomes de carbone.

10 - La chloroformyl-1 tétrahydro-1,2,3,4 quinoli-none-4 de formule générale:

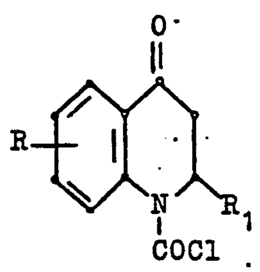

dans laquelle R représente un atome d'hydrogène ou un substituant choisi dans le groupe des atomes d'halogène et des radicaux alcoyles droits ou ramifiés contenant 1 à 4 atomes de carbone ou alcoyloxy droits ou ramifiés contenant 1 à 4 atomes de carbone et $R_1$ représente un atome d'hydrogène ou un radical alcoyle droit ou ramifié contenant 1 à 4 atomes de carbone, à l'exception des produits pour lesquels $R_1$ représente un atome d'hydrogène et R représente un atome de chlore, de brome ou de fluor en position 6.

**Revendications** pour l'Etat contractant: AT

1 - Procédé de préparation d'une tétrahydro-1,2,3,4 quinolinone-4 de formule generale:

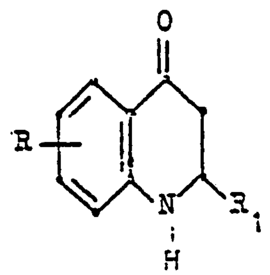

dans laquelle R représente un atome d'hydrogène ou un substituant choisi dans le groupe des atomes d'halogène et des radicaux alcoyles droits ou ramifiés contenant 1 à 4 atomes de carbone ou alcoyloxy droits ou ramifés contenant 1 à 4 1 à 4 atomes de carbone et $R_1$ représente un atome d'hydrogène ou un radical alcoyle droit ou ramifié contanant 1 à 4 atomes de carbone caractérisé en ce que l'on traite par le phosgène un acide anilino-3 propionique de formule générale:

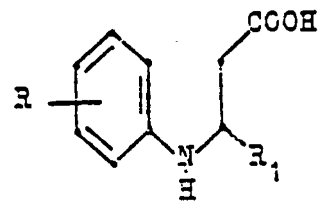

dans laquelle R et $R_1$ sont définis comme précédemment pour obtenir un produit de formule générale:

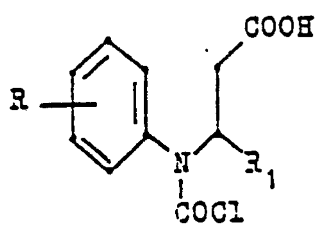

dans laquelle R et $R_1$ sont définis comme précédemment, que l'on transforme par action du phosgène en présence de diméthylformamide en chlorure d'acide de formule générale:

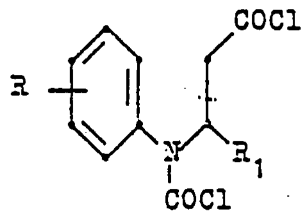

dans laquelle R et $R_1$ son définis comme précédemment, que l'on traite par un acide de Lewis dans un solvant convenable ou par un acide fort pour obtenir un produit de formule générale:

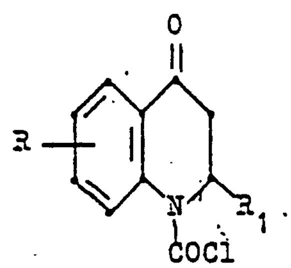

dans laquelle R et $R_1$ sont définis comme précédemment, que l'on hydrolyse en présence d'une base minérale puis isole la tétrahydro-1,2,3,4 quinolinone-4 ainsi obtenue.

2 - Procédé selon la revendication 1 caractérisé en ce que le traitement par le phosgène est effectué dans un solvant organique à une température comprise entre -10 et 150° C.

3 - Procédé selon la revendication 2 caractérisé en ce que le solvant est choisi parmi le chlorure de méthylène, le trichloro-1,1,2 éthane et le dioxanne.

4 - Procédé selon la revendication 1 caractérisé en ce que l'on utilise un acide de Lewis choisi parmi le chlorure d'aluminium, le chlorure ferrique, le chlorure de titane ou le chlorure stannique dans un solvant organique.

5 - Procédé selon la revendication 4 caractérisé en ce que le solvant organique est choisi parmi les hydrocarbures halogénés, le sulfure de carbone, les nitroalcanes, et le tétrachloroéthylène.

6 - Procédé selon la revendication 1 caractérisé en ce que l'on utilise comme acide fort l'acide sulfurique, l'acide fluorhydrique, les acides sulfoniques ou l'acide polyphosphorique.

7 - Procédé selon la revendication 1 caractérisé en ce que l'on utilise comme base la soude méthanolique.

8 - Procédé selon la revendication 1 caractérisé en ce que l'on prépare le produit de formule générale:

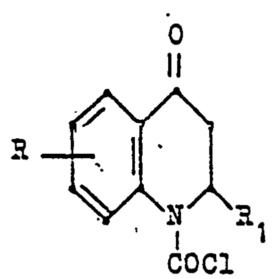

dans laquelle R et $R_1$ sont définis comme dans la revendication 1 sans isolement des produits intermédiaires.

**Patentansprüche**

für die Vertragsstaaten BE, CH, LI, DE, FR, GB, IT, LU, NL, SE

1. Verfahren zur Herstellung eines 1,2,3,4-Tetra-hydrochinolinons-4 der allgemeinen Formel

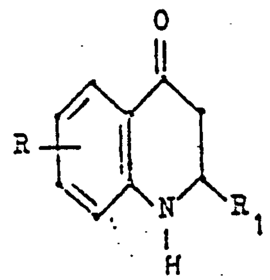

worin R ein Wasserstoffatom oder einen Substituenten bedeutet, ausgewählt aus der Gruppe der Halogenatome und gerader oder verzweigter Alkylreste mit 1 bis 4 Kohlenstoffatomen oder gerader oder verzweigter Alkoxyreste mit 1 bis 4 Kohlenstoffatomen, und $R_1$ ein Wasserstoffatom oder einen geraden oder verzweigten Alkylrest mit 1 bis 4 Kohlenstoffatomen bedeutet, dadurch gekennzeichnet, daß man eine 3-Anilino-propionsäure der allgemeinen Formel

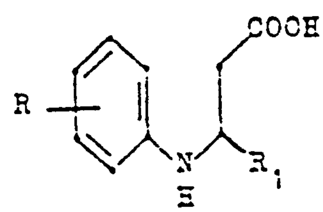

worin R und $R_1$ wie vorstehend definiert sind, mit Phosgen behandelt, um ein Produkt der allgemeinen Formel

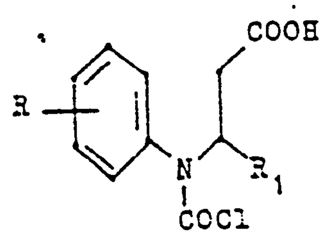

zu erhalten, worin R und $R_1$ wie vorstehend definiert sind, das man durch Einwirkung von Phosgen in Gegenwart von Dimethylformamid zum Säurechlorid der allgemeinen Formel

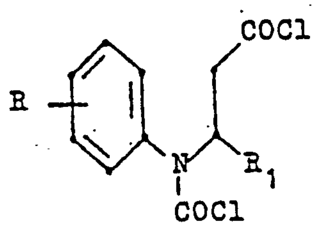

worin R und $R_1$ wie vorstehend definiert sind, umwandelt, das man miteiner Lewissäure in einem geeigneten Lösungsmittel oder mit einer starken Säure behandelt, um ein Produkt der allgemeinen Formel

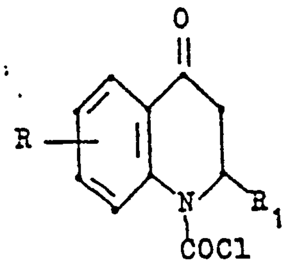

worin R und $R_1$ wie vorstehend definiert sind, zu erhalten, das man in Gegenwart einer Mineralbase hydrolysiert und das so erhaltene 1,2,3,4-Tetrahydrochinilinon-4 dann isoliert.

2. Verfahren gemäß Anspruch 1, dadurch gekennzeichnet, daß die Behandlung mit Phosgen in einem organischen Lösungsmiutel bei einer Temperatur zwischen -10 und 150°C durchgeführt wird.

3. Verfahren gemäß Anspruch 2, dadurch gekennzeichnet, daß das Lösungsmittel ausgewählt ist unter Methylenchlorid, 1,1,2-Trichlorethan und Dioxan.

4. Verfahren gemäß Anspruch 1, dadurch gekennzeichnet, daß man eine Lewissäure verwendet, ausgewählt unter Aluminiumchlorid, Ferrichlorid, Titanchlorid oder Stannichlorid in einem organischen Lösungsmittel.

5. Verfahren gemäß Anspruch 4, dadurch gekennzeichnet, daß das organische Lösungsmittel ausgewählt ist unter den halogenierten Kohlenwasserstoffen, Schwefelkohlenstoff, den Nitroalkanen und Tetrachlorethylen.

6. Verfahren gemäß Anspruch 1, dadurch gekennzeichnet, daß man als starke Säure Schwefelsäure, Fluorwasserstoffsäure, die Sulfonsäuren oder Polyphosphorsäure verwendet.

7. Verfahren gemäß Anspruch 1, dadurch gekennzeichnet, daß man als Base methanolisches Natriumhydroxid verwendet.

8. Verfahren gemäß Anspruch 1, dadurch gekennzeichnet, daß man das Produkt der allgemeinen Formel

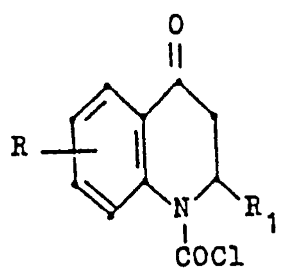

worin R und $R_1$ wie vorstehend in Anspruch 1 definiert sind, ohne Isolierung der Zwischenprodukte herstellt.

9. Das Chlorid der N-Chloroformyl-3-anilino-propion-säure der allgemeinen Formel

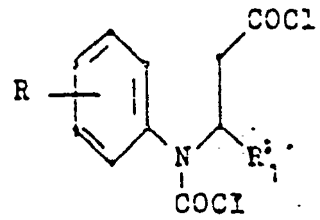

worin R ein Wasserstoffatom oder einen Substituenten, ausgewählt aus der Gruppe der Halogenatome und der geraden oder verzweigten Alkylreste mit 1 bis 4 Kohlenstoffatomen und der geraden oder verzweigten Alkoxyreste mit 1 bis 4 Kohlenstoffatomen, bedeutet und $R_1$ ein Wasserstoffatom oder einen geraden oder verzweigten Alkylrest mit 1 bis 4 Kohlenstoffatomen bedeutet.

10. Das 1°Chlorformyl-1,2,3,4-tetrahydrochinolinon-4 der allgemeinen Formel

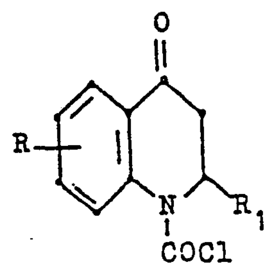

worin R ein Wasserstoffatom oder einen Substituenten, ausgewählt aus der Gruppe der Halogenatome und der geraden oder verzweigten Alkylreste mis 1 bis 4 Kohlen, stoffatomen oder der geraden oder verzweigten Alkoxyreste mit 1 bis 4 Kohlenstoffatomen, bedeutet und $R_1$ ein Wasserstoffatom oder einen geraden oder verzweigten Alkylrest mit 1 bis 4 Kohlenstoffatomen bedeutet, ausgenommen die Produkte, für die $R_1$ ein Wasserstoffatom bedeutet und R ein Chlor-, Brom- oder Fluoratom in 6-Stellung bedeutet.

**Patentansprüche**

für den Vertregsstaat AT

1.Verfahren zur Herstellung eines 1,2,3,4-Tetrahydro-chinolin-4-ons der allgemeinen Formel:

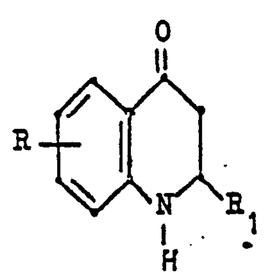

in welcher R ein Wasserstoffatom oder einen Substituenten ausgewählt aus der Gruppe von Halogenatomen und gerad- oder verzweigtkettigen Alkylgruppen mit 1 bis 4 Kohlenstoffatomen oder gerad- oder verzweigtkettigen Alkoxygruppen mit 1 bis 4 Kohlenstoffatomen bedeutet und $R_1$ ein Wasserstoffatom oder eine gerad- oder verzweigtkettige Alkylgruppe mit 1 bis 4 Kohlenstoffatomen bedeutet, dadurch gekennzeichnet, daß man eine 3-Anilinpropionsäure der allgemeinen Formel:

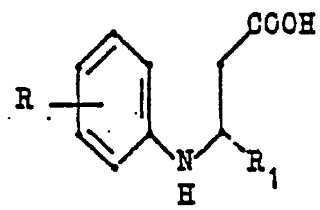

in welcher R und $R_1$ wie oben definiert sind, um ein Produkt der allgemeinen Formel:

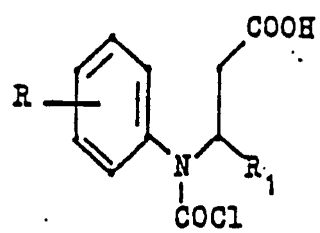

in welcher R und $R_1$ wie oben definiert sind, zu erhalten, mit Phosgen behandelt, daß man das Produkt durch Einwirkung von Phosgen in Gegenwart von Dimethylformamid in ein Säurechlorid der allgemeinen Formel:

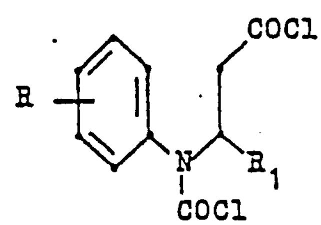

in welcher R und $R_1$ wie oben definiert sind, überführt, daß man dieses mit einer Lewis-Säure in einem geeigneten Lösungsmittel oder mit einer starken Säure behandelt, um ein Produkt der allgemeinen Formel:

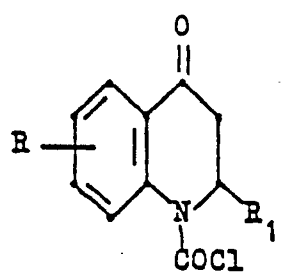

in welcher R und $R_1$ wie oben definiert sind, zu erhalten, daß man das Produkt in Gegenwart einer anorganischen Base hydrolysiert und dann das so erhaltene 1,2,3,4-Tetrahydrochinolin-4-on isoliert.

2.Verfahren nach Anspruch 1, dadurch gekennzeichnet, daß die Behandlung mit Phosgen in einem organischen Lösungsmittel bei einer Temperatur zwischen -10 und 150° C durchgeführt wird.

3. Verfahren nach Anspruch 2, dadurch gekennzeichnet, daß das Lösungsmittel ausgewählt ist aus Methylenchlorid, 1,1,2-Trichloraäthan und Dioxan.

4. Verfahren nach Anspruch 1, dadurch gekennzeichnet, daß man eine Lewis-Säure, ausgewählt aus Aluminiumchlorid, Ferrichlorid, Titanchlorid oder Zinn(IV)-chlorid in einem organischen Lösungsmittel einsetzt.

5. Verfahren nach Anspruch 4, dadurch gekennzeichnet, daß das organische Lösungsmittel ausgewählt ist aus halogenierten Kohlenwasserstoffatomen, Schwefelkohlenstoff, Nitroalkanen und Tetrachloräthylen.

6. Verfahren nach Anspruch 1, dadurch gekennzeichnet, daß man als starke Säure Schwefelsäure, Fluorwasserstoffsäure, Sulfonsäuren oder Polyphosphorsäure einsetzt.

7. Verfahren nach Anspruch 1, dadurch gekennzeichnet, daß man als Base methanolisches Natriumhydroxid einsetzt.

8. Verfahren nach Anspruch 1, dadurch gekennzeichnet, daß man das Produkt der allgemeinen Formel:

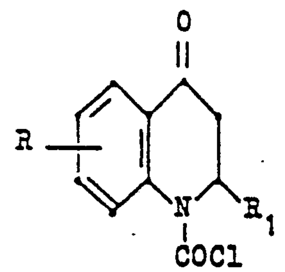

in welcher R und $R_1$ wie in Anspruch 1 definiert sind, ohne Isolierung der Zwischenprodukte herstellt.

**Claims**

for the contracting states BE CH DE FR GB IT LI LU NL SE

1.A process for the preparation of a 1,2,3,4-tetra-hydro-4-quinolinone of general formula:

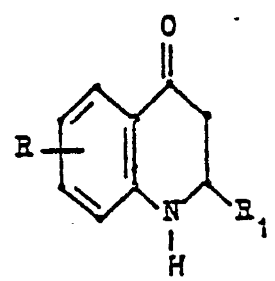

in which R denotes a hydrogen atom or a substituent chosen from the group of halogen atoms and linear or branched alkyl radicals containing 1 to 4 carbon atoms or linear or branched alkoxy radicals containing 1 to 4 carbon atoms and $R_1$ denotes a hydrogen atom or a linear or branched alkyl radical containing 1 to 4 carbon atoms, in which a 3-anilinopropionic acid of general formula:

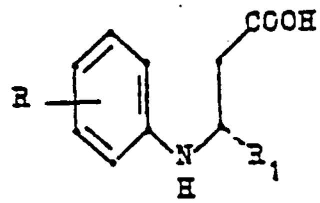

in which R and $R_1$ are defined as before is treated with phosgene to form a product of general formula:

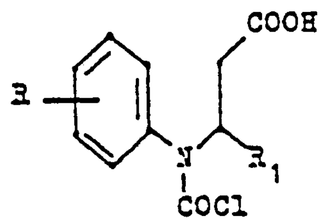

in which R and $R_1$ are defined as before, which is converted by the action of phosgene in the presence of dimethylformamide into an acid chloride of general formula:

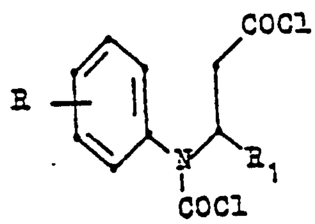

in which R and $R_1$ are defined as before, which is treated with a Lewis acid in a suitable solvent or with a strong acid to form a product of general formula:

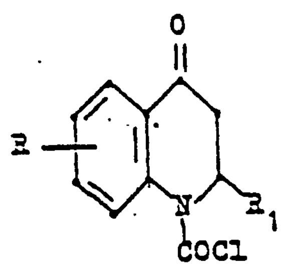

in which R and $R_1$ are defined as before, which is hydrolysed in the presence of an inorganic base, and then the 1,2,3,4-tetrahydro-4-quinolinone thus obtained is isolated.

2. The process according to claim 1, wherein the phosgene treatment is carried out in an organic solvent at a temperature of between -10 and 150°C.

3. The process according to claim 2, wherein the solvent is chosen from methylene chloride, 1,1,2-trichloroethane and dioxane.

4. The process according to claim 1, wherein a Lewis acid chosen from aluminium chloride, ferric chloride, titanium chloride or stannic chloride is used, in an organic solvent.

5. The process according to claim 4, wherein the organic solvent is chosen from halogenated hydrocarbons, carbon disulphide, nitroalkanes and tetrachloroethylene.

6. The process according to claim 1, wherein sulphuric acid, hydrofluoric acid, a sulphonic acid or polyphospoic acid is used as the strong acid.

7. The process according to claim 1, wherein methanolic sodium hydroxide is used as a base.

8. The process according to claim 1, wherein the product of general formula:

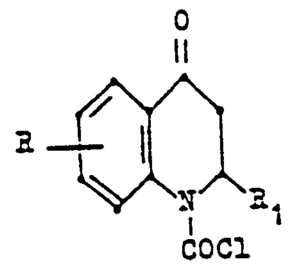

in which R and $R_1$ are defined as in claim 1, is prepared without isolation of the intermediate products.

9. The chloride of N-chloroformyl-3-anilinopropionic acid of general formula:

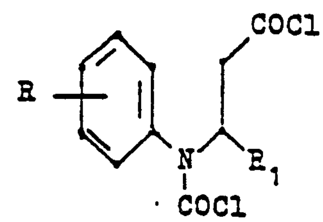

in which R denotes a hydrogen atom or a substituent chosen from the group of halogen atoms and linear or branched alkyl radicals containing 1 to 4 carbon atoms and linear or branched alkoxy radicals containing 1 to 4 carbon atoms and $R_1$ denotes a hydrogen atom or a linear or branched alkyl radical containing 1 to 4 carbon atoms.

10. 1-Chloroformyl-1,2,3,4-tetrahydro-4-quinolinone of general formula:

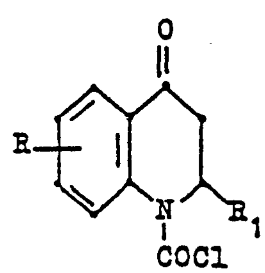

in which R denotes a hydrogen atom or a substituent chosen from the group of halogen atoms and linear or branched alkyl radicals containing 1 to 4 carbon atoms or linear or branched alkoxy radicals containing 1 to 4 carbon atoms and $R_1$ denotes a hydrogen atom or a linear or branched alkyl radical containing 1 to 4 carbon atoms, with the exception of the products in which $R_1$ denotes a hydrogen atom and R denotes a chlorine, bromine or fluorine atom in the 6 position.

## Claims

for the contracting state AT

1. A process for the preparation of a 1,2 3 4-tetra-hydro-4-quinolinone of general formula:

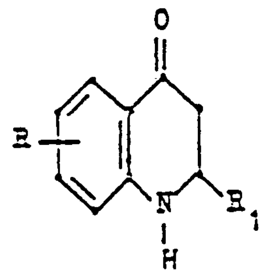

in which R denotes a hydrogen atom or a substituent chosen from the group of halogen atoms and linear or branched alkyl radicals containing 1 to 4 carbon atoms or linear or branched alkoxy radicals containing 1 to 4 carbon atoms and $R_1$ denotes a hydrogen atom or a linear or branched alkyl radical containing 1 to 4 carbon atoms, in which a 3-anilinopropionic acid of general formula:

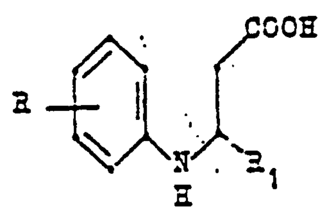

in which R and $R_1$ are defined as before is treated with phosgene to form a product of general formula:

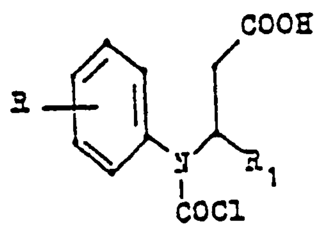

in which R and $R_1$ are define as before, which is converted by the action of phosgene in the presence of dimethylformamide into an acid chloride of general formula:

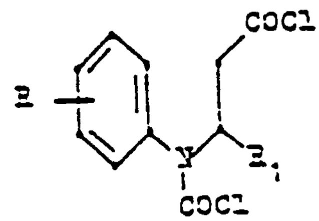

in which R and $R_1$ are defined as before, which is treated with a Lewis acid in a suitable solvent or with a strong acid to form a product of general formula:

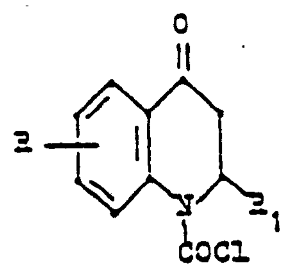

in which R and $R_1$ are defined as before, which is hydrolysed in the presence of an inorganic base, and then the 1,2,3,4-tetrahydro-4-quinolinone thus obtained is isolated.

2. The process according to claim 1, wherein the phosgene treatment is carried out in an organic solvent at a temperature of between -10 and 150°C.

3. The process according to claim 2, wherein the solvent is chosen from methylene chloride, 1,1,2-trichloroethane and dioxane.

4. The process according to claim 1, wherein a Lewis acid chosen from aluminium chloride, ferric chloride, titanium chloride or stannic chloride is used, in an organic solvent.

5. The process according to claim 4, wherein the organic solvent is chosen from halogenated hydrocarbons, carbon disulphide, nitroalkanes and tetrachloroethylene.

6. The process according to claim i, wherein sulphuric acid, hydrofluoric acid, a sulphonic acid or polyphosphoric acid is used as the strong acid.

7. The process according to claim 1, wherein methanolic sodium hydroxide is used as a base.

8. The process according to claim 1, wherein the product of general formula:

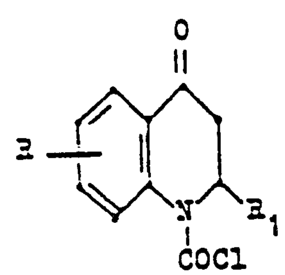

in which R and $R_1$ are defined as in claim 1, is prepared without isolation of the intermediate products.